(19) Europäisches Patentamt
European Patent Office
Office européen des brevets

(11) EP 4 647 422 A1

(12) # EUROPEAN PATENT APPLICATION
published in accordance with Art. 153(4) EPC

(43) Date of publication:
**12.11.2025 Bulletin 2025/46**

(21) Application number: **24738706.1**

(22) Date of filing: **03.01.2024**

(51) International Patent Classification (IPC):
***C07D 207/267*** (2006.01) ***C08G 63/06*** (2006.01)
***C08J 11/12*** (2006.01) ***C08J 11/28*** (2006.01)

(52) Cooperative Patent Classification (CPC):
**C07D 207/267; C08G 63/06; C08J 11/12; C08J 11/28**

(86) International application number:
**PCT/KR2024/000118**

(87) International publication number:
**WO 2024/147636 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.01.2023 KR 20230001264**

(71) Applicant: **CJ CheilJedang Corporation**
**Seoul 04560 (KR)**

(72) Inventors:
- **YOU, Youngsu**
  **Seoul 04560 (KR)**
- **JEON, Jinwoo**
  **Seoul 04560 (KR)**
- **SEO, Jang-Woo**
  **Seoul 04560 (KR)**
- **SHIN, Hun Yi**
  **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **METHOD FOR PRODUCING N-METHYL-2-PYRROLIDONE USING POLYHYDROXYALKANOATE, AND N-METHYL-2-PYRROLIDONE PREPARED THEREFROM**

(57) The present invention relates to a method for producing N-methyl-2-pyrrolidone using polyhydroxyalkanoate and to N-methyl-2-pyrrolidone produced therefrom, the production method comprising: (1) preparing polyhydroxyalkanoate; (2) reacting the polyhydroxyalkanoate and an amine compound at 110-180°C to produce an acyclic amide compound; and (3) heating the acyclic amide compound.

[Fig. 1]

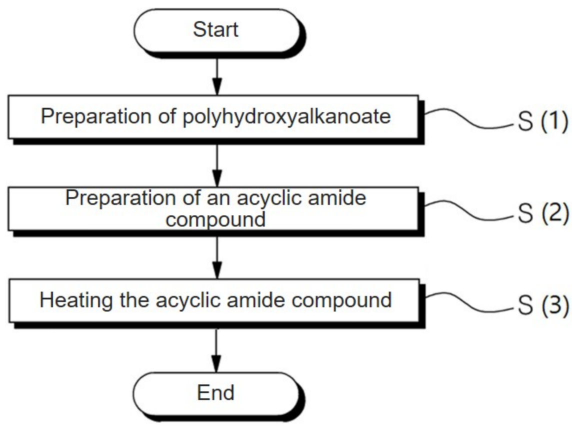


EP 4 647 422 A1

## Description

### Technical Field

The present disclosure relates to a process for preparing methylpyrrolidone (N-methyl-2-pyrrolidone, NMP), a chemical substance with high added value, using polyhydroxyalkanoate.

### Background Art

Bio-refinery is a technology for preparing biofuel (energy), power, heat, and high-value-added chemicals from biomass through biological or chemical conversion processes. This bio-refinery technology is similar to an oil-refinery technology for producing fuel and petrochemical products from crude oil as an oil resource. Currently, the oil refinery has environmental pollution problems due to greenhouse gases inevitably generated during the process, and limitations exist due to the depletion of oil resources. Thus, research has been conducted to prepare chemicals with high-added value using biomass rather than petroleum resources.

Polyhydroxyalkanoate (PHA), as a biomass, is a storage material within microbial cells and is attracting attention as a biodegradable material that can be completely decomposed. In recent years, technologies for producing high-value-added chemicals or intermediates through chemical depolymerization of polyhydroxyalkanoate have been developed. For example, methylpyrrolidone (N-methyl-2-pyrrolidone, NMP) is prepared using polyhydroxyalkanoate.

Methylpyrrolidone is chemically stable and has excellent thermal resistance; thus, it is used as a starting material for various organic syntheses that require an inert medium or an organic solvent. In addition, methylpyrrolidone has high polarity and solubility; thus, it is used as a cleaning solvent or an additive in the electrical and electronic fields and the paint industry. In addition, methylpyrrolidone is used as a binder component for coating cathode materials in the secondary battery industry, which is a key element of the ESS industrial technology that can store produced energy. If methylpyrrolidone, which is widely used in various fields, can be prepared using polyhydroxyalkanoate as a biomass, it can reduce environmental pollution and become a substitute for petroleum resources that are to be depleted.

However, the process for preparing methylpyrrolidone using polyhydroxyalkanoate currently available is complex, and the yield of methylpyrrolidone is not satisfactory.

[Prior Art Document]

[Patent Document]

(Patent Document 1) Korean Laid-open Patent Publication No. 2011-0058002

### Disclosure of Invention

### Technical Problem

As described above, conventionally, there have been limitations in producing methylpyrrolidone in an environmentally friendly manner and with a high yield. Specifically, 1,4-butanediol (BDO) derived from petroleum resources is converted to a $\gamma$-butyrolactone (GBL) intermediate in the presence of a metal catalyst, which is then reacted with an alkylamine to produce methylpyrrolidone in a conventional process. However, this entails the depletion of petroleum resources, causing environmental pollution, and the use of metal catalysts, which reduces economic feasibility.

Accordingly, attempts have been made to produce methylpyrrolidone using polyhydroxyalkanoate as a biomass in order to reduce the depletion of petroleum resources and produce methylpyrrolidone in an environmentally friendly manner. However, this has problems such as process complexity and reduced yield of methylpyrrolidone.

The present inventors have conducted various studies to solve these problems. As a result, it has been discovered that methylpyrrolidone can be produced in a high yield through a relatively simple process by optimizing the reactants and reaction conditions used in the depolymerization reaction of polyhydroxyalkanoate.

Accordingly, an object of the present disclosure is to provide a process for preparing methylpyrrolidone using polyhydroxyalkanoate in an environmentally friendly and economical manner and with a high yield.

In addition, another object of the present disclosure is to provide methylpyrrolidone prepared by the above preparation process.

### Solution to Problem

In order to accomplish the above object, the present disclosure provides a process for preparing methylpyrrolidone that comprises (1) preparing a polyhydroxyalkanoate; (2) reacting the polyhydroxyalkanoate and an amine compound at 110 to 180°C to prepare an acyclic amide compound; and (3) heating the acyclic amide compound.

According to an embodiment of the present disclosure, in step (1), the polyhydroxyalkanoate may comprise a repeat unit derived from 4-hydroxybutyrate (4HB).

According to another embodiment of the present disclosure, in step (2), the amine compound may comprise a compound selected from the group consisting of monomethylamine, an aqueous monomethylamine solution, dimethylamine, ammonia, and ammonium water.

According to another embodiment of the present disclosure, in step (2), the polyhydroxyalkanoate and the amine compound may react at an equivalent ratio of 1:1.01 to 2.5.

According to another embodiment of the present disclosure, in step (2), the reaction time of the polyhydroxyalkanoate and the amine compound may be 10 hours or less.

According to another embodiment of the present disclosure, in step (2), the acyclic amide compound may comprise a compound selected from the group consisting of 4-hydroxy-N-methylbutanamide, 4-hydroxybutanamide, and 4-hydroxy-N-(2-hydroxyethyl)butanamide.

According to another embodiment of the present disclosure, in step (3), the heating temperature of the acyclic amide compound may be 200°C or higher.

According to another embodiment of the present disclosure, in step (3), the initial pressure for heating the acyclic amide compound may be 1 to 80 bar.

According to another embodiment of the present disclosure, in step (3), the difference ($P_2$ - $P_1$) between the initial pressure ($P_1$) for heating the acyclic amide compound and the equilibrium pressure ($P_2$) 1 hour after heating may be 15 bar or more.

According to another embodiment of the present disclosure, in step (3), the heating time of the acyclic amide compound may be 1 to 8 hours.

According to another embodiment of the present disclosure, in step (2), the conversion rate of the polyhydroxyalkanoate may be 80% or more, and the selectivity of the acyclic amide compound may be 90% or more.

According to another embodiment of the present disclosure, in step (3), the conversion rate of the acyclic amide compound may be 90% or more.

According to another embodiment of the present disclosure, the yield of methylpyrrolidone, when prepared by the above preparation process, is 90% or more.

Meanwhile, in order to accomplish the above object, the present disclosure provides methylpyrrolidone prepared by the above preparation process.

## Advantageous Effects of Invention

Since the present disclosure produces methylpyrrolidone using polyhydroxyalkanoate, which is a biomass, as a starting material instead of petroleum resources, methylpyrrolidone can be produced in an environmentally friendly manner while reducing the depletion of petroleum resources. In addition, the present disclosure can produce methylpyrrolidone economically and in a high yield by optimizing the depolymerization reaction of polyhydroxyalkanoate to produce methylpyrrolidone.

## Brief Description of Drawing

Fig. 1 is a flowchart showing the preparation process of methylpyrrolidone according to an embodiment of the present disclosure.

## Best Mode for Carrying out the Invention

Hereinafter, the present disclosure will be described in detail. The present disclosure is not limited to the disclosures given below, but it may be modified into various forms as long as the gist of the disclosure is not changed.

In the present specification, the term "comprising" is intended to specify a particular characteristic, region, step, process, element, and/or component. It does not exclude the presence or addition of any other characteristic, region, step, process, element and/or component, unless specifically stated to the contrary.

All numbers and expressions related to the quantities of components, reaction conditions, and the like used herein are to be understood as being modified by the term "about" unless otherwise indicated.

The present disclosure provides a process for preparing methylpyrrolidone using polyhydroxyalkanoate as a biomass and methylpyrrolidone prepared by the process. The present disclosure is characterized in that methylpyrrolidone can be prepared in an environmentally friendly and economical manner with a high yield by optimizing the reactants and reaction conditions used in the depolymerization process of polyhydroxyalkanoate. Hereinafter, this will be described

in detail.

**Process for preparing methylpyrrolidone**

The process for preparing methylpyrrolidone according to the present disclosure comprises (1) preparing a polyhydroxyalkanoate; (2) reacting the polyhydroxyalkanoate and an amine compound at 110 to 180°C to prepare an acyclic amide compound; and (3) heating the acyclic amide compound.

Hereinafter, each step will be described with reference to Fig. 1.

Step (1): Preparation of a polyhydroxyalkanoate

According to the present disclosure, step (1) is to prepare a polyhydroxyalkanoate, which is a biomass.

Polyhydroxyalkanoate is a natural thermoplastic polyester polymer that accumulates within microbial cells. It has physical properties similar to those of synthetic biodegradable polymers derived from petroleum, such as polybutylene adipate terephthalate (PBAT), polybutylene succinate (PBS), polybutylene succinate terephthalate (PBST), and polybutylene succinate adipate (PBSA), and has excellent characteristics in terms of biodegradability and biocompatibility.

The polyhydroxyalkanoate may be obtained by cell disruption using a mechanical method or a physical method, or it may be obtained by cell disruption using a nonmechanical method or a chemical method. Specifically, the polyhydroxyalkanoate may be obtained through microbial cell disruption using at least one selected from the group consisting of ultrasonic disruption, high-pressure disruption, and milling disruption.

The ultrasonic disruption may be carried out for 10 to 60 minutes at an energy level of 20 Hz or higher. Specifically, the ultrasonic disruption may be carried out for 10 to 60 minutes, 15 to 55 minutes, or 20 to 50 minutes at an energy level of 60 Hz or less, 50 Hz or less, or 40 Hz or less.

The high-pressure disruption may be carried out for 1 to 60 minutes at a pressure of 10 bar or more. Specifically, the high-pressure disruption may be carried out for 1 to 60 minutes, 2 to 60 minutes, or 3 to 60 minutes at a pressure of 10 bar or more, 20 bar or more, or 50 bar or more.

The milling disruption may be carried out for 1 to 60 minutes, 2 to 60 minutes, or 3 to 60 minutes using a colloid mill, a bead mill, or a ball mill.

More specifically, the polyhydroxyalkanoate may be obtained through enzyme-catalyzed polymerization of one or more monomers (monomer repeat units) within microbial cells, followed by cell disruption.

The polyhydroxyalkanoate obtained in this manner may have a purity of 90% or more, specifically, 92% or more, 94% or more, 96% or more, 98% or more, 99% or more, or 99.9% or more (e.g., 90 to 100%, 95 to 100%, or 98 to 99.5%), but it is not limited thereto.

The polyhydroxyalkanoate may have a weight average molecular weight ($M_w$) of 10,000 to 1,200,000 g/mole, 50,000 to 1,000,000 g/mole, 100,000 to 900,000 g/mole, 150,000 to 800,000 g/mole, 200,000 to 700,000 g/mole, or 250,000 to 600,000 g/mole, but it is not limited thereto.

The polyhydroxyalkanoate may have a glass transition temperature ($T_g$) of -45 to 80°C, -35 to 70°C, -30 to 60°C, -25 to 50°C, -20 to 30°C, -15 to 15°C, or -15 to 0°C, but it is not limited thereto.

The crystallization temperature ($T_c$) of the polyhydroxyalkanoate may, or may not, be measured. Specifically, the crystallization temperature ($T_c$) of the polyhydroxyalkanoate may not be measured or may be 70 to 120°C, 75 to 120°C, 75 to 115°C, 75 to 110°C, or 80 to 110°C, but it is not limited thereto.

The melting temperature ($T_m$) of the polyhydroxyalkanoate may, or may not, be measured. Specifically, the melting temperature ($T_m$) of the polyhydroxyalkanoate may not be measured or may be 100 to 170°C, 110 to 150°C, 115 to 145°C, or 120 to 140°C, but it is not limited thereto.

The polyhydroxyalkanoate may have a decomposition temperature ($T_d$) of 140 to 310°C, 160 to 290°C, 190 to 260°C, or 220 to 230°C, but it is not limited thereto. For example, the decomposition temperature ($T_d$) may be 140 to 160°C, 190 to 220°C, 230 to 260°C, or 290 to 310°C.

The polyhydroxyalkanoate may have a polydispersity index (PDI) of 1.0 or more, 1.2 or more, 1.5 or more, 1.8 or more, 1.9 or more, or 2.0 or more, and 5.0 or less, 4.0 or less, 3.0 or less, 2.9 or less, 2.8 or less, 2.7 or less, 2.6 or less, or 2.5 or less, but it is not limited thereto.

The polyhydroxyalkanoate may comprise a repeat unit (a 4HB repeat unit) derived from 4-hydroxybutyrate (4HB). Specifically, the polyhydroxyalkanoate may be in the form of a polymer consisting only of the 4HB repeat unit, or in the form of a copolymer comprising the 4HB repeat unit and a repeat unit different from the 4HB repeat unit. More specifically, the polyhydroxyalkanoate may be selected from the group consisting of poly-4-hydroxybutyrate (P4HB) and poly(3-hydroxybutyrate-co-4-hydroxybutyrate) (P3HB-co-4HB), but it is not limited thereto. As the polyhydroxyalkanoate is one of the above substances, it has excellent reactivity with an amine compound, whereby it can be readily converted to an acyclic amide compound. As a result, methylpyrrolidone can be prepared in an environmentally friendly manner and with a high yield.

Meanwhile, the crystallinity of the polyhydroxyalkanoate (PHA) may be adjusted depending on the content of the 4HB repeat unit. It may be classified into semi-crystalline PHA (scPHA) and amorphous PHA (aPHA).
Specifically, the semi-crystalline PHA (scPHA) may have a content of a 4HB repeat unit of 0.1 to 30% by weight, 1 to 28% by weight, 3 to 26% by weight, 5 to 25% by weight, 8 to 23% by weight, 10 to 20% by weight, or 10 to 15% by weight, based on the total weight of the polyhydroxyalkanoate (PHA). In addition, the amorphous PHA (aPHA) may have a content of a 4HB repeat unit of 15 to 60% by weight, 20 to 58% by weight, 25 to 55% by weight, 30 to 53% by weight, 35 to 50% by weight, 40 to 49% by weight, or 45 to 48% by weight, based on the total weight of the polyhydroxyalkanoate (PHA).
The polyhydroxyalkanoate (PHA) may be composed of the semi-crystalline PHA (scPHA) alone, the amorphous PHA (aPHA) alone, or a mixture thereof. Specifically, it may be amorphous PHA (aPHA). For example, when the polyhydroxyalkanoate (PHA) is poly(3-hydroxybutyrate-co-4-hydroxybutyrate) (P3HB-co-4HB), it may be amorphous P3HB-co-4HB with a content of a 4HB repeat unit of 15 to 60% by weight.

Step (2): Preparation of an acyclic amide compound

According to the present disclosure, step (2) is to react the polyhydroxyalkanoate with an amine compound at a specific temperature to prepare an acyclic amide compound in a high yield.
The reaction temperature of the polyhydroxyalkanoate and the amine compound may be 110 to 180°C. Specifically, the reaction temperature may be 110 to 175°C, 113 to 170°C, 113 to 165°C, 115 to 160°C, 115 to 155°C, 118 to 150°C, 118 to 145°C, or 120 to 140°C, but it is not limited thereto.
The reaction time of the polyhydroxyalkanoate and the amine compound may be 10 hours or less. Specifically, the reaction time may be 9 hours or less, 8 hours or less, 7 hours or less, or 6 hours or less (e.g., 1 to 10 hours, 2 to 10 hours, 3 to 10 hours, or 6 to 10 hours), but it is not limited thereto.
In addition, the reaction ratio between the polyhydroxyalkanoate and the amine compound may be an equivalent ratio of 1:1.01 to 2.5. Specifically, the reaction ratio may be an equivalence ratio of 1:1.05 to 2.4, an equivalence ratio of 1:1.08 to 2.3, an equivalence ratio of 1:1.1 to 2.25, or an equivalence ratio of 1:1.1 to 2.2, but it is not limited thereto.
As the reaction temperature, reaction time, and reaction ratio each satisfy the above ranges, polyhydroxyalkanoate is readily converted to an acyclic amide compound. This allows methylpyrrolidone to be produced in a high yield.
The amine compound may not be particularly limited as long as it is a compound capable of depolymerizing the polyhydroxyalkanoate. Specifically, the amine compound may comprise a compound selected from the group consisting of monomethylamine, an aqueous monomethylamine solution, dimethylamine, ammonia, and ammonium water. As the amine compound comprises the above compound, the selectivity and yield of the acyclic amide compound for conversion to methylpyrrolidone can be increased.
The amine compound may be in the form of a gas or an aqueous solution (concentration: 25 to 40% by weight).
In addition, the amine compound may have a vapor density of 0.52 to 1.65, specifically, 0.58 to 1.61, a boiling point of -40 to -2°C, specifically, -33 to -6°C, and a pH of 10.8 to 11.8, specifically, 11.2 to 11.5, but it is not limited thereto.
Meanwhile, the acyclic amide compound obtained by the reaction of the polyhydroxyalkanoate and the amine compound may comprise a compound selected from the group consisting of 4-hydroxy-N-methylbutanamide, 4-hydroxybutanamide, and 4-hydroxy-N-(2-hydroxyethyl)butanamide, but it is not limited thereto.
The acyclic amide compound may have a boiling point of 100 to 110°C or 150 to 160°C at 0.2 Torr and a boiling point of 230 to 250°C at normal pressure, but it is not limited thereto.
As step (2) is carried out, the present disclosure has a high conversion rate of the polyhydroxyalkanoate (the rate at which polyhydroxyalkanoate is converted to an acyclic amide compound) and a high selectivity of the acyclic amide compound. This allows methylpyrrolidone to be produced in a high yield.
Specifically, according to the present disclosure, the conversion rate of the polyhydroxyalkanoate may be 80% or more, 82% or more, 84% or more, 86% or more, 88% or more, or 90% or more (e.g., 80 to 100%, 83 to 100%, 85 to 100%, or 90 to 99.9%), but it is not limited thereto.
In addition, according to the present disclosure, the selectivity of the acyclic amide compound may be 90% or more, 92% or more, 94% or more, 96% or more, 98% or more, or 99% or more (e.g., 90 to 100%, 92 to 100%, 94 to 99.9%, or 97 to 99.9%), but it is not limited thereto.
According to the present disclosure, the yield of the acyclic amide compound may be 40% or more, 50% or more, 60% or more, 70% or more, 80% or more, 85% or more, or 90% or more (e.g., 50 to 100%, 70 to 100%, 85 to 99.9%, or 90 to 99.9%), but it is not limited thereto.

Step (3): Heating of the acyclic amide compound

According to the present disclosure, step (3) is to heat the acyclic amide compound under relatively high temperature and pressure conditions. Specifically, the acyclic amide compound is heated under high-temperature and high-pressure conditions to cause a condensation cyclization reaction to produce methylpyrrolidone.

The heating temperature of the acyclic amide compound may be 200°C or higher, specifically, 210°C or higher, 220°C or higher, 230°C or higher, 240°C or higher, or 250°C or higher (e.g., 200 to 300°C, 210 to 290°C, 220 to 280°C, 230 to 275°C, 235 to 270°C, 240 to 260°C, or 245 to 255°C), but it is not limited thereto.

In addition, the initial pressure (setting pressure) for heating the acyclic amide compound may be 1 to 80 bar, specifically, 1 to 75 bar, 1 to 70 bar, 1 to 65 bar, 3 to 60 bar, 5 to 60 bar, or 10 to 60 bar, but it is not limited thereto.

In addition, the difference ($P_2$ - $P_1$) between the initial pressure ($P_1$) for heating the acyclic amide compound and the equilibrium pressure ($P_2$) 1 hour after heating may be 15 bar or more. Specifically, the pressure difference ($P_2$ - $P_1$) may be 16 bar or more, 17 bar or more, 18 bar or more, 20 bar or more, 23 bar or more, or 25 bar or more, and 100 bar or less, 90 bar or less, 80 bar or less, or 70 bar or less (e.g., 15 to 100 bar, 17 to 95 bar, 19 to 90 bar, 20 to 85 bar, 25 to 80 bar, or 27 to 75 bar), but it is not limited thereto.

In addition, the heating time of the acyclic amide compound may be 1 to 8 hours, specifically, 1 to 7 hours, 2 to 6 hours, 2 to 5 hours, or 3 to 4 hours, but it is not limited thereto.

As the heating temperature, initial pressure, pressure difference ($P_2$ - $P_1$), and heating time are each within the above ranges, the reaction stability of the acyclic amide compound is secured, and the acyclic amide compound is readily converted to methylpyrrolidone. This allows methylpyrrolidone to be produced in a high yield.

If methylpyrrolidone is produced as in step (3), specifically, if step (3) is a step of heating the acyclic amide compound to produce methylpyrrolidone, the present disclosure has a high conversion rate of the acyclic amide compound (the rate at which the acyclic amide compound is converted to methylpyrrolidone) and a high selectivity of methylpyrrolidone. This allows methylpyrrolidone to be produced in a high yield.

Specifically, according to the present disclosure, the conversion rate of the acyclic amide compound may be 90% or more, 92% or more, 95% or more, 97% or more, 99% or more, or 99.9% or more (e.g., 90 to 100%, 92 to 100%, 95 to 100%, 98 to 100%, or 99.9 to 100%), but it is not limited thereto.

In addition, according to the present disclosure, the selectivity of methylpyrrolidone may be 85% or more, 88% or more, 90% or more, 92% or more, 95% or more, 97% or more, 99% or more, or 99.9% or more (e.g., 85 to 100%, 88 to 100%, 90 to 100%, 95 to 99.9%, or 99 to 99.9%), but it is not limited thereto.

In addition, according to the present disclosure, the yield of methylpyrrolidone may be 90% or more, 91% or more, 92% or more, 94% or more, 96% or more, 98% or more, 99% or more, or 99.9% or more (e.g., 90 to 100%, 91 to 100%, 92 to 100%, 95 to 99.9%, or 99 to 99.9%), but it is not limited thereto.

## Methylpyrrolidone

Methylpyrrolidone according to the present disclosure is prepared by the preparation process described above. That is, as the present disclosure prepares methylpyrrolidone using polyhydroxyalkanoate, methylpyrrolidone with high purity can be provided in a high yield and in an environmentally friendly and economical manner.

Specifically, according to the present disclosure, methylpyrrolidone may have a high purity of at least 87% or more, 90% or more, 92% or more, 95% or more, or 98% or more (e.g., 87 to 99.9%, 87 to 99%, or 87 to 98%).

In addition, methylpyrrolidone may comprise less than 0.1% by weight of 2-pyrrolidone as a by-product.

Methylpyrrolidone according to the present disclosure can be advantageously used in various fields such as electrical and electronic fields, energy fields (e.g., secondary batteries), chemical fields (e.g., organic synthesis and organic solvents), or paint fields (e.g., detergents and additives).

## Mode for the Invention

Hereinafter, the present disclosure will be described in detail with reference to Examples, but the scope of the present disclosure is not limited to the Examples.

### [Reagents and analysis equipment]

Chloroform (> 99.5%; Daejeong Chemical and Metals), methanol (EP grade; Daejeong Chemical and Metals), ethanol (GR grade; Duksan Pure Chemicals), acetonitrile (HPLC grade; BURDICK&JACKSON), and monomethylamine (aqueous solution having a concentration of 40% by weight; Samchun Pure Chemical) were used reagents in the Examples, Comparative Examples, and Test Examples.

In addition, high-performance liquid chromatography (HPLC) and gas chromatography were used as analysis equipment.

Specifically, 1260 infinity equipment from Agilent Technologies was used as the HPLC equipment, and Capcell Pak C18 MG (4.6 mm × 250 mm × 5 μm, P/N 92635) from Osaka Soda was used as the column. Here, the autosampler temperature was set at 15°C, the column temperature was set at 35°C, tertiary distilled water containing 0.2% phosphoric acid and acetonitrile (ACN) was used as a mobile phase solvent, and the analysis was performed with a gradient elution at

a mobile phase flow rate of 1 ml/min.

GC (Agilent Technologies, 8890) equipped with a DB-WAX (60 m × 250 μm × 0.25 μm) column was used as the GC equipment. Here, the inlet temperature was set at 250°C, and the flow rate was 1 ml/min. In addition, the flame ionization detector (FID) temperature was set at 300°C for the analysis.

**[Example 1]**

Preparation of poly-4-hydroxybutyrate (P4HB) (purification)

300 ml of bacterial broth containing poly-4-hydroxybutyrate (P4HB) was added to a centrifuge and centrifuged at 3,000 rpm for 5 minutes to remove the supernatant. Next, 300 ml of primary distilled water was added, the cells were disentangled through vortexing, and centrifugation was performed again. Subsequently, the supernatant was removed again, 150 g of chloroform was added, and it was stirred at 45°C for 2 hours using a mechanical stirrer. Next, the supernatant was removed, and the chloroform layer only was slowly added to an excess of ethanol (EtOH) to allow P4HB to precipitate. Thereafter, EtOH and chloroform were removed through drying to obtain a P4HB precipitate. The obtained P4HB precipitate was a white solid, and its purity was confirmed to be 99%.

Preparation of 4-hydroxy-N-methylbutanamide (4HBA)

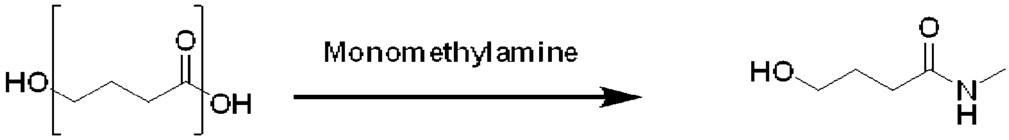


P4HB

4-Hydroxy-*N*-methylbutanamide

0.2 g (1 equivalent) of the P4HB obtained through the purification above and 0.2 g (1.1 equivalents) of monomethylamine (MMA, aqueous solution having a concentration of 40% by weight) were added to a 48 ml screw cap-sealed tube and stirred at 300 rpm to prepare a mixture. Subsequently, the mixture was reacted at 120°C for 6 hours to prepare 4HBA.

**[Examples 2 to 10 and Comparative Examples 1 to 9]**

4HBA was prepared following the same procedure as in Example 1, except that the reaction temperature and time of the mixture of P4HB and MMA and the equivalent ratio of P4HB and MMA were changed as shown in Table 1 below.

**[Test Example 1]**

In each of Examples 1 to 10 and Comparative Examples 1 to 9, the P4HB remaining after the preparation of 4HBA was separated using a paper filter and then weighed to calculate the conversion rate of P4HB. The results are shown in Table 1 below. In addition, the selectivity and yield of 4HBA in the reaction solution were analyzed using high-performance liquid chromatography (HPLC) and calculated using the following equations. The results are shown in Table 1 below.

4HBA selectivity (%) = (number of moles of 4HBA produced / number of moles of 4HB in converted P4HB) × 100 [Equation 1]

[Equation 2]

4HBA yield (%) = P4HB conversion rate × 4HBA selectivity

[Table 1]

| | Equivalent ratio of P4HB:MMA | Reaction temp. (°C) of P4HB and MMA | Reaction time (hr) of P4HB and MMA | Conversion rate (%) of P4HB | Selectivity (%) of 4HBA | Yield (%) of 4HBA |
|---|---|---|---|---|---|---|
| Ex. 1 | 1:1.1 | 120 | 6 | 100 | 80.5 | 80.5 |
| Ex. 2 | 1:1.1 | 120 | 10 | 95.0 | 99.2 | 94.2 |
| Ex. 3 | 1:1.1 | 140 | 6 | 74.2 | 99.2 | 73.6 |
| Ex. 4 | 1:1.1 | 140 | 10 | 100 | 99.9 | 99.9 |
| Ex. 5 | 1:2.2 | 120 | 3 | 90.8 | 98.5 | 89.4 |
| Ex. 6 | 1:2.2 | 120 | 6 | 100 | 99.9 | 99.9 |
| Ex. 7 | 1:2.2 | 120 | 10 | 100 | 99.9 | 99.9 |
| Ex. 8 | 1:2.2 | 140 | 3 | 100 | 99.9 | 99.9 |
| Ex. 9 | 1:2.2 | 140 | 6 | 100 | 99.9 | 99.9 |
| Ex. 10 | 1:2.2 | 140 | 10 | 100 | 99.9 | 99.9 |
| C. Ex. 1 | 1:1.1 | 25 | 6 | 25.8 | 98.1 | 25.3 |
| C. Ex. 2 | 1:1.1 | 25 | 10 | 45 | 87.1 | 39.2 |
| C. Ex. 3 | 1:1.1 | 90 | 6 | 7.2 | 68.1 | 4.9 |
| C. Ex. 4 | 1:1.1 | 90 | 10 | 71.1 | 89.2 | 63.4 |
| C. Ex. 5 | 1:2.2 | 25 | 3 | 50.2 | 81.9 | 41.1 |
| C. Ex. 6 | 1:2.2 | 25 | 6 | 79.5 | 97.7 | 77.7 |
| C. Ex. 7 | 1:2.2 | 25 | 10 | 72 | 99.9 | 71.9 |
| C. Ex. 8 | 1:2.2 | 90 | 3 | 37.1 | 56.9 | 21.1 |
| C. Ex. 9 | 1:2.2 | 90 | 6 | 64.2 | 98.9 | 63.5 |

Referring to Table 1 above, in the present disclosure, as the mixture of P4HB and MMA was reacted at a temperature of 110°C or higher (Examples 1 to 10), the P4HB conversion rate and 4HBA selectivity increased, so that 4HBA was obtained in a high yield. In contrast, when the mixture of P4HB and MMA was reacted at a low temperature of 90°C or lower, the P4HB conversion rate and 4HBA selectivity decreased, so that the yield of 4HBA decreased.

**[Example 11]**

Preparation of methyl-2-pyrrolidone (NMP)

4HBA obtained in Example 4 was dried in a vacuum oven (BF-60VO, Biofree) at 50°C for 16 hours, 20 g of which was added to a high-temperature and high-pressure reactor (R-101 Model High Pressure Bomb System, Chemesis). After the initial pressure ($P_1$) was adjusted to normal pressure (1 bar), it was then reacted at 250°C for 3 hours. Here, the equilibrium pressure ($P_2$) was measured after 1 hour of reaction. Upon completion of the reaction, the product was diluted with methanol and recovered to obtain N-methyl-2-pyrrolidone (NMP).

**[Examples 12 to 15]**

NMP was prepared following the same procedure as in Example 11, except that the initial pressure was adjusted by injecting nitrogen as shown in Table 2 below.

**[Comparative Example 10]**

NMP was prepared following the same procedure as in Example 11, except that 4HBA obtained in Comparative Example 6 was used.

**[Test Example 2]**

In each of Examples 11 to 15 and Comparative Example 10, the 4HBA remaining after the preparation of NMP was separated using a paper filter and then weighed to calculate the conversion rate of 4HBA. The results are shown in Table 2 below. In addition, the selectivity and yield of NMP were analyzed using gas chromatography (GC) and calculated using the following equations. The results are shown in Table 2 below.

NMP selectivity (%) = (number of moles of NMP produced / number of moles of 4HBA converted) × 100 [Equation 3]

[Equation 4]

$$\text{NMP yield (\%)} = \text{4HBA conversion rate} \times \text{NMP selectivity}$$

[Table 2]

| | Reaction temp. (°C) | Reaction time (hr) | Initial pressure (bar) | Equilibrium pressure (bar) | Conversion rate (%) of 4HBA | Selectivity (%) of NMP | Yield (%) of NMP |
|---|---|---|---|---|---|---|---|
| Ex. 11 | 250 | 3 | 1 | 20 | 99.9 | 92.2 | 92.1 |
| Ex. 12 | 250 | 3 | 10 | 38 | 100 | 99.9 | 99.9 |
| Ex. 13 | 250 | 3 | 40 | 90 | 100 | 99.9 | 99.9 |
| Ex. 14 | 250 | 3 | 60 | 130 | 100 | 99.9 | 99.9 |
| Ex. 15 | 250 | 3 | 70 | 145 | 98.5 | 83.1 | 81.9 |
| C. Ex. 10 | 250 | 3 | 1 | 20 | 96.4 | 81.3 | 78.4 |

Referring to Table 2 above, in the present disclosure, as 4HBA was reacted under relatively high temperature and pressure conditions, the 4HBA conversion rate and NMP selectivity were high, so NMP was obtained in a high yield.

**Claims**

1. A process for preparing methylpyrrolidone, which comprises:

   (1) preparing a polyhydroxyalkanoate;
   (2) reacting the polyhydroxyalkanoate and an amine compound at 110 to 180°C to prepare an acyclic amide compound; and
   (3) heating the acyclic amide compound.

2. The process for preparing methylpyrrolidone of claim 1, wherein, in step (1), the polyhydroxyalkanoate comprises a repeat unit derived from 4-hydroxybutyrate (4HB).

3. The process for preparing methylpyrrolidone of claim 1, wherein, in step (2), the amine compound comprises a compound selected from the group consisting of monomethylamine, an aqueous monomethylamine solution, dimethylamine, ammonia, and ammonium water.

4. The process for preparing methylpyrrolidone of claim 1, wherein, in step (2), the polyhydroxyalkanoate and the amine compound react at an equivalent ratio of 1:1.01 to 2.5.

5. The process for preparing methylpyrrolidone of claim 1, wherein, in step (2), the reaction time of the polyhydroxyalkanoate and the amine compound is 10 hours or less.

6. The process for preparing methylpyrrolidone of claim 1, wherein, in step (2), the acyclic amide compound comprises a compound selected from the group consisting of 4-hydroxy-N-methylbutanamide, 4-hydroxybutanamide, and 4-hydroxy-N-(2-hydroxyethyl)butanamide.

7. The process for preparing methylpyrrolidone of claim 1, wherein, in step (3), the heating temperature of the acyclic amide compound is 200°C or higher.

8. The process for preparing methylpyrrolidone of claim 1, wherein, in step (3), the initial pressure for heating the acyclic amide compound is 1 to 80 bar.

9. The process for preparing methylpyrrolidone of claim 1, wherein, in step (3), the difference ($P_2$ - $P_1$) between the initial pressure ($P_1$) for heating the acyclic amide compound and the equilibrium pressure ($P_2$) 1 hour after heating is 15 bar or more.

10. The process for preparing methylpyrrolidone of claim 1, wherein, in step (3), the heating time of the acyclic amide compound is 1 to 8 hours.

11. The process for preparing methylpyrrolidone of claim 1, wherein, in step (2), the conversion rate of the polyhydroxyalkanoate is 80% or more, and the selectivity of the acyclic amide compound is 90% or more.

12. The process for preparing methylpyrrolidone of claim 1, wherein, in step (3), the conversion rate of the acyclic amide compound is 90% or more.

13. The process for preparing methylpyrrolidone of claim 1, wherein the yield of methylpyrrolidone is 90% or more.

14. Methylpyrrolidone, which is prepared by the preparation process according to any one of claims 1 to 13.

[Fig. 1]

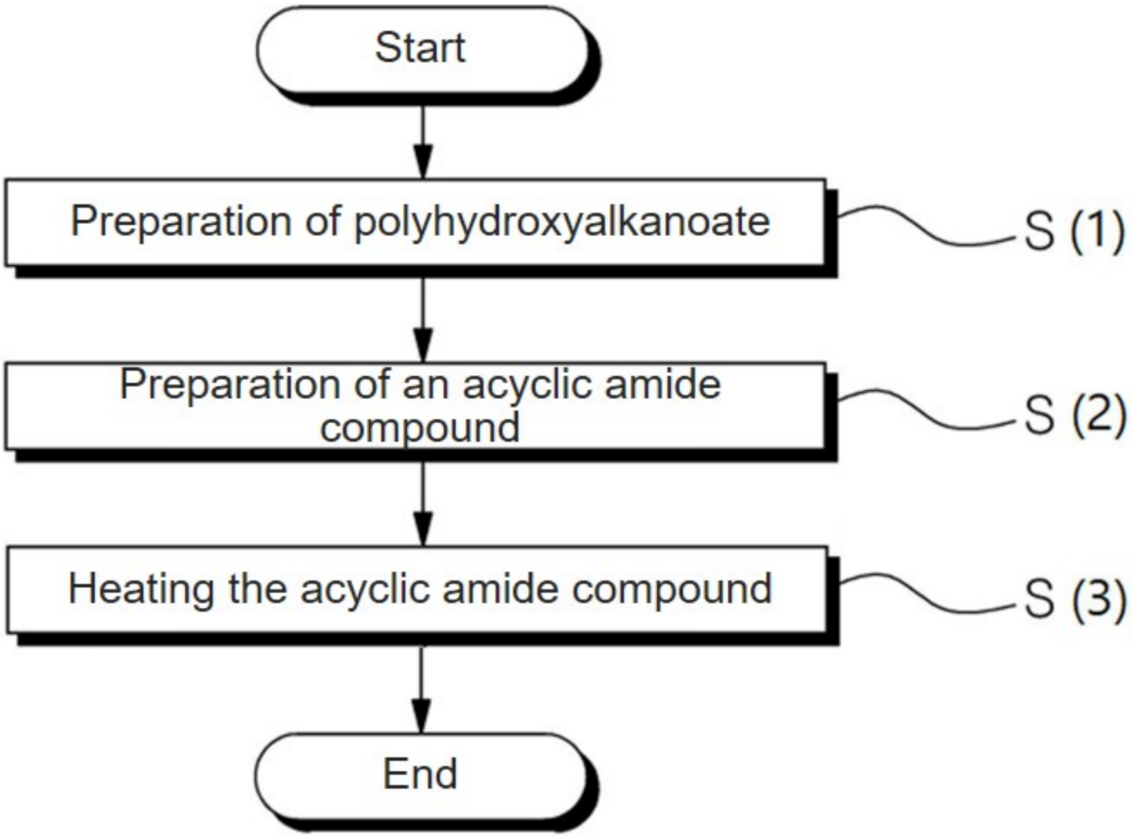
Start
Preparation of polyhydroxyalkanoate
S (1)
Preparation of an acyclic amide compound
S (2)
Heating the acyclic amide compound
S (3)
End

**INTERNATIONAL SEARCH REPORT**

International application No.
**PCT/KR2024/000118**

**A. CLASSIFICATION OF SUBJECT MATTER**

**C07D 207/267**(2006.01)i; **C08G 63/06**(2006.01)i; **C08J 11/12**(2006.01)i; **C08J 11/28**(2006.01)i

According to International Patent Classification (IPC) or to both national classification and IPC

**B. FIELDS SEARCHED**

Minimum documentation searched (classification system followed by classification symbols)

C07D 207/267(2006.01); C07C 67/02(2006.01); C07C 69/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리하이드록시알카노에이트(polyhydroxyalkanoate, PHA), 비고리형 아마이드(acyclic amide), 메틸 피롤리돈(methyl pyrrolidone), 온도(temperature)

**C. DOCUMENTS CONSIDERED TO BE RELEVANT**

| Category* | Citation of document, with indication, where appropriate, of the relevant passages | Relevant to claim No. |
|---|---|---|
| X | WO 03-051813 A1 (METABOLIX, INC. et al.) 26 June 2003 (2003-06-26)<br>See pages 13-18, 24 and 25. | 1-14 |
| A | KR 10-2000-0035624 A (MITSUBISHI CHEMICAL CORPORATION) 26 June 2000 (2000-06-26)<br>See claims 1, 4 and 8. | 1-14 |
| A | JP 2000-256312 A (TONEN CHEM. CORP.) 19 September 2000 (2000-09-19)<br>See claim 1; and paragraphs [0026]-[0030]. | 1-14 |
| A | JP 2000-229940 A (TONEN CHEM. CORP.) 22 August 2000 (2000-08-22)<br>See paragraphs [0015]-[0017]. | 1-14 |
| A | BOYANDIN, A. N. et al. Aminolysis of poly-3-hydroxybutyrate in N,N-dimethylformamide and 1,4-dioxane and formation of functionalized oligomers. Polymers. 2022, vol. 14, thesis no. 5481, inner pp. 1-15.<br>See pages 3, 4 and 11. | 1-14 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2024** | **12 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/000118**

| Patent document cited in search report | Publication date (day/month/year) | Patent family member(s) | Publication date (day/month/year) |
|---|---|---|---|
| WO 03-051813 A1 | 26 June 2003 | AU 2002-357355 A1 | 30 June 2003 |
| | | US 2003-0144551 A1 | 31 July 2003 |
| | | US 2003-0158274 A1 | 21 August 2003 |
| | | US 2003-0158441 A1 | 21 August 2003 |
| | | US 2003-0162851 A1 | 28 August 2003 |
| | | US 2005-0101760 A1 | 12 May 2005 |
| | | US 2005-0182235 A1 | 18 August 2005 |
| | | US 6844447 B2 | 18 January 2005 |
| | | US 6897338 B2 | 24 May 2005 |
| | | US 6933404 B2 | 23 August 2005 |
| | | US 7001969 B2 | 21 February 2006 |
| | | US 7166743 B2 | 23 January 2007 |
| | | US 7230144 B2 | 12 June 2007 |
| KR 10-2000-0035624 A | 26 June 2000 | EP 1004577 A1 | 31 May 2000 |
| | | EP 1004577 B1 | 30 June 2004 |
| | | JP 2000-219675 A | 08 August 2000 |
| | | JP 4036580 B2 | 23 January 2008 |
| | | US 2002-0035270 A1 | 21 March 2002 |
| | | US 6429316 B1 | 06 August 2002 |
| JP 2000-256312 A | 19 September 2000 | JP 2000-256313 A | 19 September 2000 |
| | | KR 10-2001-0006761 A | 26 January 2001 |
| JP 2000-229940 A | 22 August 2000 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20110058002